# EUROPÄISCHE PATENTANMELDUNG

(11) **EP 0 852 136 A1**
(43) Veröffentlichungstag der Anmeldung: **08.07.1998**
(21) Anmeldenummer: 97810851.2
(22) Anmeldetag: 11.11.1997
(51) Int. Cl.: A61K 7/13

(54) **Verfahren zum Färben von keratinhaltigen Fasern**

(30) Priorität: 19.11.1996 CH 2852/96
(71) Anmelder: Ciba Specialty Chemicals Holding Inc., 4057 Basel (CH)
(72) Erfinder: Möckli, Peter, 4124 Schönenbuch (CH)

(57) **Zusammenfassung**

Zum Färben von keratinhaltigen Fasem, insbesondere von menschlichen Haar, verwendet man Farbstoffe der im Anspruch 1 angegebenen Formel (1).

## Beschreibung

Die vorliegende Erfindung betrifft ein Verfahren zum Färben von keratinhaltigen Fasern, insbesondere menschlichem Haar, mit kationischen Farbstoffen.

Der weitaus grösste Teile aller Haarfärbungen wird auch heute noch mit sogenannten "Oxidationsfarben" durchgeführt, wobei kleine, farblose Vorläuferverbindungen auf das Haar aufgebracht und dann durch einen Oxidationsprozess zu grösseren, farbigen Verbindungen verbunden werden. Obwohl auf diese Art die dauerhaftesten ("permanenten") Färbungen erzielt werden, werden doch in zunehmendem Masse Bedenken laut über mögliche toxikologische Risiken sowohl der eingesetzten Substanzen wie auch der durch die Oxidation entstehenden Zwischen- und Endprodukte, deren genaue Zusammensetzung praktisch nicht kontrollierbar ist. Weitere Nachteile sind die relativ komplizierte Anwendung und vor allem auch die Haarschädigungen durch die eingesetzten Chemikalien.

Bei den übrigen, sogenannten "semipermanenten" und "temporären" Färbungen werden fertige Farbstoffe eingesetzt und zwar in erster Linie ungeladene Dispersionfarbstoffe und in geringerem Umfang wasserlösliche Säurefarbstoffe. Kationische Farbstoffe spielen dagegen nur eine ganz geringe Rolle. Wie aus der Bezeichnung "semipermanent" und "temporär" schon hervorgeht, weisen diese Färbungen nur ein mittleres bis schlechtes Echtheitsniveau auf. lnsbesondes die kationischen Farbstoffe stehen im Ruf schlechter Hydrolyse- und Lichtbeständigkeit sowie ungleichmässiger Anfärbung der Haare, z.B. zwichen Wurzel und Spitze (siehe: John F. Corbett: "The chemistry of Hair-care Products," JSCD August 1976, S. 290).

Darüber hinaus besitzen die bekannten kationischen Farbstoffe einen ungenügenden Aufbau, d.h. auch bei erhöhter Einsatzmenge kann eine bestimmte, relativ geringe, Farbstärke nicht überschritten werden. So ist es bei spielsweise nicht möglich, mit den wichtigsten kationischen Haarfarbstoffen Basic Yellow 57, Basic Red 76, Basic Blue 99, Basic Brown 16 und Basic Brown 17, die in der Praxis verwendet werden, eine tiefe Schwarzfärbung zu erreichen. Relativ dunkle Naturhaare lassen sich aus dem gleichen Grund mit diesen Farbstoffen nur noch schlecht nuancieren.

Es sind auch bereits Anthrachinonfarbstoffe mit kationischen Substituenten als Haarfarbstoffe beschrieben, z.B. in J. Soc. Cosm. Chem. 18, 705-713 (1967) und JSDC Nov. 1968, 556-560. Auch diese vermögen jedoch nicht allen Anforderungen zu genügen.

Es wurde nun gefunden, dass überraschenderweise kationische Farbstoffe der nachfolgend angegebenen Formel (1) sich hervorragend zum Färben von Haaren eignen. Man kann mit ihnen auf einfache Weise und unter haarschonenden Bedingungen Färbungen mit ausgezeichneten Licht-, Shampoonier- und Reibechtheiten erreichen. Aufgrund ihrer ausserordentlich reinen Nuancen erweitern sie ausserdem den Kreis der möglichen Misch-Nuancen beträchtlich, insbesondere in Richtung der besonders wichtigen Blautöne.

Gegenstand der vorliegenden Erfindung ist daher ein Verfahren zum Färben von keratinhaltigen Fasern, welches dadurch gekennzeichnet ist, dass man die Fasern mit einem Farbstoff der Formel 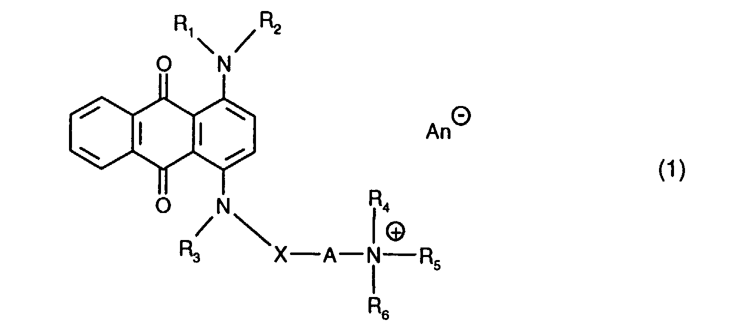 behandelt, worin
R₁ und R₃ unabhängig voneinander je Wasserstoff oder Methyl,
R₂ Wasserstoff oder C₁-C₃-Alkyl,
R₄ und R₅ unabhängig voneinander je C₁-C₂-Alkyl,
R₆ C₁-C₃-Alkyl oder Benzyl,
X die direkte Bindung oder 1,4-Phenylen,
A C₁-C₄-Alkylen und
An^{⊖} ein farbloses Anion bedeutet.

Unter Alkylresten sind erfindungsgemäss generell geradkettige oder verzweigte Alkylgruppen zu verstehen. In Frage kommen Methyl, Ethyl, n- und iso-Propyl.

Das Anion ist im allgemeinen durch das Herstellungsverfahren vorgegeben. Bevorzugte Anionen sind Chlorid, Sulfat, Hydrogensulfat, Methylsulfat, Phosphat, Formiat, Acetat oder Lactat.

Unter den Farbstoffen der Formel (1) sind diejenigen besonders bevorzugt, bei denen R₁ und R₂ jeweils Wasserstoff bedeuten.

Femer sind solche Farbstoffe der Formel (1) bevorzugt, bei denen R₄ und R₅ jeweils Ethyl oder vor allem Methyl bedeuten.

R6 bedeutet vorzugsweise Ethyl oder Methyl oder, falls X die direkte Bindung ist, Benzyl.

A ist vorzugsweise Methylen, Ethylen oder vor allem n-Propylen.

Die Farbstoffe der Formel (1) sind bekannt oder können auf an sich bekannte Art und Weise hergestellt werden.

Die erfindungsgemässen Verfahren eignen sich zum Färben von Pelzen sowie von tierischem und menschlichem Haar, insbesondere von lebendem menschlichem Haar und Haaren von Haustieren. Infolge der hohen Affinität und der guten Wasserlöslichkeit der verwendeten Farbstoffe kann man je nach Haarqualität bereits bei Raumtemperatur aus wässrigen Lösungen ohne jegliche Hilfsmittel färben. Üblicherweise erfolgen die Färbungen bei einer Temperatur zwischen etwa 20 und 40 °C.

Es können aber auch alle für kationische Farbstoffe üblichen Hilfsmittel, die beim Färben von Haaren Verwendung finden, eingesetzt werden, beispielsweise Benetzungsmittel, Quellmittel, Durchdringungsmittel und/oder Parfums. Ausserdem können die Farbstoffe in Shampoos, Cremes, Gelées oder Pasten eingearbeitet werden. Solche kosmetischen Formulierungen zum Färben von Haaren, enthaltend mindestens einen Farbstoff der oben angegebenen Formel (1) sowie weitere Hilfsmittel und bilden einen weiteren Gegenstand der vorliegenden Erfindung.

Ein besonderer Vorteil der erfindungsgemäss verwendeten Farbstoffe zum Färben von Haaren besteht darin, dass man wegen des guten Aufbaus dieser Farbstoffe Färbungen nach dem Trichromieprinzip herstellen kann, wenn man sie zusammen mit einem geigneten gelben und einem geeigneten roten Farbstoff einsetzt.

Geeignete gelbe und rote Farbstoffe sind insbesondere diejenigen, die in der WO-95/01772 und WO-95/15144 beschrieben sind.

Mit geeigneten Mischungen dieser Farbstoffe kann man praktisch alle Nuancen erzielen. Zudem ist eine bessere Voraussage der erhaltenen Nuancen möglich, was bei den blauen sog. "Oxidationsfarbstoffen" wegen der wechselnden Zusammensetzung der Endprodukte nicht der Fall ist.

Unter Einsatz colorimetrischer Messverfahren kann man ausserdem auch bei natürlichem, nicht gebleichtem Haar vorausbestimmte Farbnuancen erhalten unter Berücksichtigung der Eigenfarbe des Haares, indem man deren Gelb-, Rot- und Blauanteil bestimmt und diesen bei der Rezeptur der angestrebten Nuance in Abzug bringt. Dies ist mit den bisher in der Praxis gebrauchten Haarfarbstoffen nicht durchführbar.

Die erhaltenen Färbungen sind reib-, wasser-, wasch- und lichtecht und beständig gegen Dauerverformungsmittel, wie z.B. Thioglykolsäure.

Die folgenden Beispiele veranschaulichen die Erfindung. Teile bedeuten Gewichtsteile und Prozente Gewichtsprozente. Die Temperaturen sind in Grad Celsius angegeben.

Beispiel 1: Eine auf Tresse genähte Strähne eines hellblonden, natürlichen, nicht behandelten Menschenhaares wird bei 25°C während 5 Minuten auf übliche Weise mit einer Farbstoffemulsion gefärbt, enthaltend 0,1 % des blauen Farbstoffes der Formel 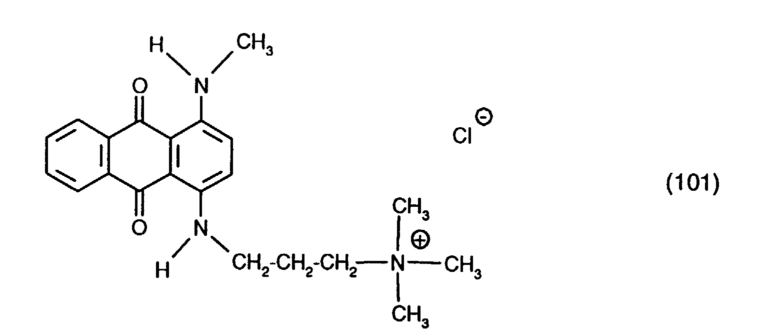
3,5% Cetearylalkohol
1,0% Ceteareth 80
0,5% Glycerylmono-di-stearat
3,0% Stearamid DEA
1,0% Stearamphopropylsulfonat
0,5% Polyquaternium-6 und
Wasser ad 100%.

Danach wird das Haar gründlich mit Wasser gespült und an der Luft getrocknet. Es resultiert eine intensive Blaufärbung. Licht-, Shampoonier- und Friktionsechtheiten der erfindungsgemässen Färbung sind hervorragend.

Beispiel 2: Ersetzt man den Farbstoff im Beispiel 1 durch die gleiche Menge des Farbstoffes der Formel 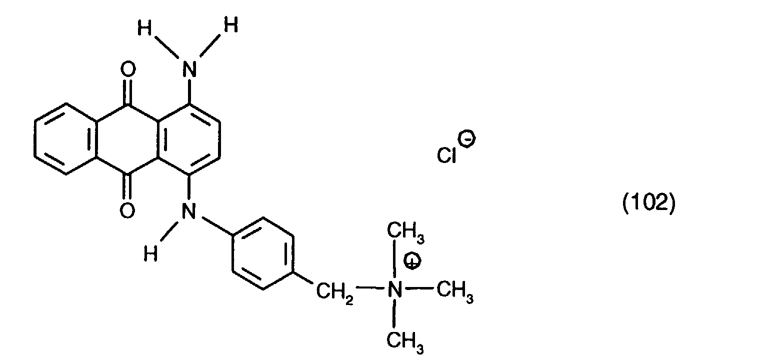 und verfährt sonst gleich, so erhält man ebenfalls eine intensiv blaue Färbung mit ebenfalls hervorragenden Echtheiten.

Beispiel 3: Eine 1 %-ige Lösung des Farbstoffes der Formel 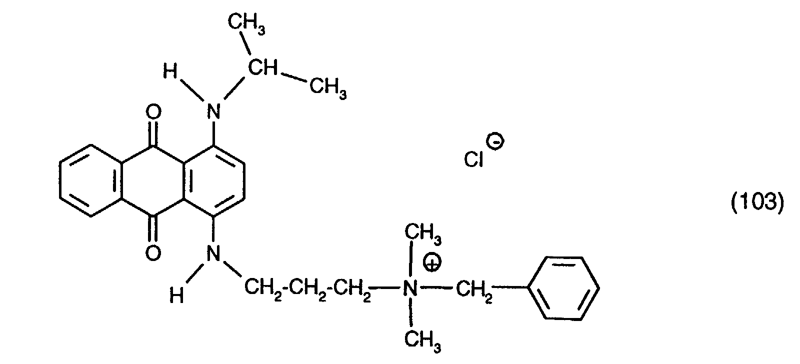 in einer Tensidgrundlage, bestehend aus 10% Cocoamphoglycinat und 90% Wasser, wird auf chinesisches, gebleichtes Yakhaar während 5 Minuten bei 25°C appliziert, anschliessend wird das Haar gut ausgespült und an der Luft getrocknet. Man erhält eine intensiv blaue Färbung mit guter Lichtechtheit.

## Patentansprüche

1. Verfahren zum Färben von keratinhaltigen Fasem, dadurch gekennzeichnet, dass man die Fasern mit einem Farbstoff der Formel 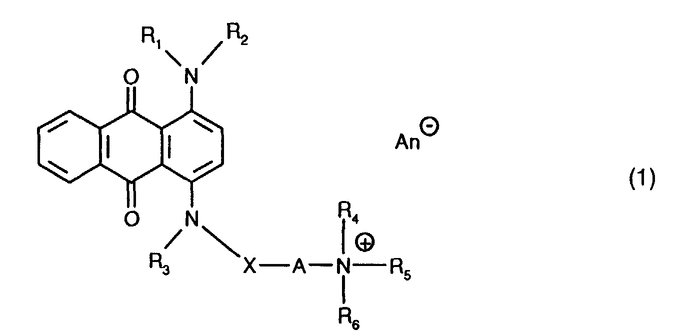 behandelt, worin
R₁ und R₃ unabhängig voneinander je Wasserstoff oder Methyl,
R₂ Wasserstoff oder C₁-C₃-Alkyl,
R₄ und R₅ unabhängig voneinander je C₁-C₂-Alkyl,
R₆ C₁-C₃-Alkyl oder Benzyl,
X die direkte Bindung oder 1,4-Phenylen,
A C₁-C₄-Alkylen und
An^{⊖} ein farbloses Anion bedeutet.

2. Verfahren gemäss Anspruch 1, dadurch gekennzeichnet, dass man einen Farbstoff der Formel (1) verwendet, worin R₁ und R₂ jeweils Wasserstoff bedeuten.

3. Verfahren gemäss einem der Ansprüche 1 oder 2, dadurch gekennzeichnet, dass man einen Farbstoff der Formel (1) verwendet, worin R₄ und R₅ jeweils Ethyl oder Methyl bedeuten.

4. Verfahren gemäss Anspruch 3, dadurch gekennzeichnet, dass man einen Farbstoff der Formel (1) verwendet, worin R₄ und R₅ jeweils Methyl bedeuten.

5. Verfahren gemäss einem der Ansprüche 1 bis 4, dadurch gekennzeichnet, dass man einen Farbstoff der Formel (1) verwendet, worin R₆ Methyl oder Ethyl oder, falls X die direkte Bindung ist, Benzyl bedeutet.

6. Verfahren gemäss einem der Ansprüche 1 bis 5, dadurch gekennzeichnet, dass man einen Farbstoff der Formel (1) verwendet, worin X die direkte Bindung und A Methylen, Ethylen oder n-Propylen bedeutet.

7. Verfahren gemäss Anspruch 6, dadurch gekennzeichnet, dass man einen Farbstoff der Formel (1) verwendet, worin X die direkte Bindung und A n-Propylen bedeutet.

8. Verfahren gemäss einem der Ansprüche 1 bis 5, dadurch gekennzeichnet, dass man einen Farbstoff der Formel (1) verwendet, worin X 1,4-Phenylen A Methylen bedeutet.

9. Verfahren gemäss einem der Ansprüche 1 bis 8, dadurch gekennzeichnet, dass man die Fasem mit einer Mischung aus einem blauen kationischen Farbstoff der Formel (1) sowie einem gelben und einem roten kationischen Farbstoff behandelt.

10. Verfahren gemäss einem der Ansprüche 1 bis 9 zum Färben von lebendem menschlichem Haar.

11. Verfahren gemäss einem der Ansprüche 1 bis 9 zum Färben von Haaren von Haustieren.

12. Verfahren zum Färben von Haaren an lebenden Tieren und Menschen, dadurch gekennzeichnet, dass eines der Verfahren gemäss den Ansprüchen 1 bis 11 unter Einsatz colorimetrischer Messverfahren zur Erzielung vorausbestimmbarer Farbnuancen angewendet wird.

13. Kosmetische Formulierung zur Haarfärbung, enthaltend mindestens einen der Farbstoffe der Formel (1) gemäss Anspruch 1 sowie weitere Hilfsmittel.

14. Verfahren zum Färben von Haaren an lebenden Tieren und Menschen, dadurch gekennzeichnet, dass man eine Mischung von mindestens einem blauen Farbstoff der Formel (1) und einem gelben und einem roten kationischen Farbstoff unter Einsatz colorimetrischer Messverfahren zur Erzielung vorausbestimmbarer Farbnuancen anwendet.
